# EUROPEAN PATENT APPLICATION

(11) **EP 1 334 986 A2**
(43) Date of publication of application: **13.08.2003**
(21) Application number: 03002824.5
(22) Date of filing: 07.02.2003
(51) Int. Cl.: C08F 283/06, C08F 283/00, C08F 2/08, C11D 3/37, C11D 3/00, C11D 17/06, C11D 17/00, A61K 7/48, A61K 7/42

(54) **Hydrophobe-amine graft copolymer**

(30) Priority: 08.02.2002 US 72402
(71) Applicant: National Starch and Chemical Investment Holding Corporation, New Castle, Delaware 19720 (US)
(72) Inventor: Rodrigues, Klein A., Signal Mountain, Tennessee 37377 (US); Crossman, Martin, Hixson, Tennessee 37343 (US); Goodson, Susanne H., Warrington, Pennsylvania 18976 (US)
(74) Representative: Held, Stephan, Dipl.-Chem. Dr.rer.nat.

(57) **Abstract**

The present invention relates to a graft polymer having a backbone moiety and at least one moiety grafted onto the backbone moiety where the backbone and grafted moieties are a hydrophobe and an amine or amide. The graft copolymer may have either a hydrophobic backbone or a mixed amine/hydrophobic backbone with a grafted amine or amide moiety; or an amine functional backbone with a grafted hydrophobic moiety. The backbone may be polymeric or non-polymeric. The graft copolymer may be used to deliver an active ingredient onto a substrate. The active ingredient may be associated, coated or encapsulated by the graft copolymer. The graft copolymer can be designed to have a pH triggerable solubility, providing a means to control the release of an active ingredient at a desired pH level.

## Description

### FIELD OF THE INVENTION

This invention relates to a graft copolymer containing an amine or an amide and a hydrophobe. The polymer is useful as a delivery system in aqueous media. The polymers are particularly useful in detergent and cleaning applications.

### BACKGROUND OF THE INVENTION

Most substrates, such as fabrics, wood, ceramics, paper, clothing, tend to be anionic. Molecules having a cationic functionality are attracted to these anionic substrates. Because of this known interaction, molecules have been designed with cationic functionality to facilitate attraction to these anionic substrates. One useful example is the attachment of an amine functionality to a polymer to facilitate the attachment of the polymer to a textile or fabric.

WO 00/56849 describes a linear copolymer that could have amine or quaterntized amine functionality useful in a detergent composition in providing dye maintenance protection to fabrics.

WO 99/14297 describes a detergent composition containing surfactants and a mixture of linear amine based polymers.

WO 00/49122 describes polyamine backbone cross-linked polymers that may or may not have a grafted moiety. Grafting is used to elongate the polyamine chains with nitrogen functional monomers.

Unfortunately amine functional polymers tend to adversely affect detergent properties such as detergency, anti-redeposition, and related cleaning properties.

There is a need for a polymeric compound having a cationic functionality (to facilitate attraction to an anionic substrate) which will not adversely effect cleaning ability by also attracting dirt and dyes to the substrate. Surprisingly, it has been found that graft copolymers containing a balance of an amine and a hydrophobic functionality overcomes this problem, making them useful in a wide variety of applications. While not being bound to any theory, it is believed that the hydrophobe serves to minimize the interaction of the amine functionality with surfactants typically found in a detergent. Additionally a graft polymer is advantageous over the linear polymers currently used in the art, since grafting allows control over the amount of functional groups in the polymer and allows for an incorporation of a wider variety of functional groups. The copolymer grafting mechanism provides a means to balance the water solubility (amine groups) and the water insolubility (hydrophobic groups) on the polymer molecule, to optimize desired properties. Grafting also provides a means of incorporating molecules and functionalities containing no double bonds into a polymer, permitting the incorporation of groups unavailable for polymerization into a typical linear copolymer.

### SUMMARY OF THE INVENTION

The present invention is directed to a graft polymer comprising a backbone moiety and at least one moiety grafted onto the backbone moiety, wherein said backbone and grafted moieties comprise a hydrophobe and an amine or amide. The graft copolymer may have either a hydrophobic backbone or a mixed amine/hydrophobic backbone with a grafted amine or amide moiety; or an amine functional backbone with a grafted hydrophobic moiety. The backbone may be polymeric or non-polymeric.

The invention is also directed to a composition containing a amine or amide/hydrophobe graft copolymer and a detergent ingredient.

The invention is also directed to a substrate having thereon an amine or amide/hydrophobe graft copolymer.

The invention is further directed to a method for the delivery an active ingredient comprising:
a) coating or encapsulating an active ingredient with the graft copolymer of claim 1;
b) introducing said coated or encapsulated active ingredient into an aqueous environment; and
c) decreasing the pH of the aqueous environment to solubilize the graft copolymer, thereby releasing the active ingredient.

The method applies to the delivery of both water-soluble additives, and non-water soluble moieties to a surface from an aqueous medium.

The polymers of this invention are particularly useful at providing fabric care properties to detergent and fabric softener compositions.

### DETAILED DESCRIPTION OF THE INVENTION

The present invention relates to a graft polymer containing an amine or amide and a hydrophobe in which the amine functionality and hydrophobicity are balanced to optimize the polymer utility. The molecule may consist of either a hydrophobic backbone with one or more amine or amide-functional polymer arms grafted onto it, or may be an amine-functional backbone with one or more hydrophobic polymer arms grafted onto it.

The backbone may consist of either a hydrophobe, an amine, or a combination of the two. The backbone must contain some functionality, or a mixture of functionalities, enabling one to graft onto said backbone. The hydrophobic backbone may be polymeric or may be non-polymeric. In the case of a polymeric backbone, the functionality may be present in the monomers used to form the polymeric backbone, or the polymer formed may be functionalized after polymerization.

A polymeric hydrophobic backbone may consist of polymers known in the art and made by means known in the art including homo- and co-polymers; linear, branched, and star polymers; random and block polymers; and crosslinked polymers. Monomers useful in forming the polymeric backbone include, but not limited to (meth)acrylates, maleates, (meth)acrylamides, vinyl esters, itaconates, styrenics, unsaturated hydrocarbons and acrylonitrile, nitrogen functional monomers, vinyl esters, alcohol functional monomers, glycols, epoxides unsaturated hydrocarbons, and (meth)acrylates. Preferred hydrophobic monomers are vinyl monomers and acrylate monomers, especially C₁₋₁₂ acrylates and methacrylates, Furthermore, the polymeric backbone may be a polymer not derived from a ethelenically unsaturated monomer, such as for example poly(propylene glycol), poly(butylene glycol), poly(ethylene imine), ethoxylated poly(ethylene imine) and other such polymers.

The backbone hydrophobe can also be non-polymeric and consist of molecules such as glycols and cationic, anionic, non-ionic and amphoteric surfactants. Preferred surfactants include alcohol ethoxylate surfactants.

The hydrophobic backbone could also be a natural polymer, such as a polysaccharide, cellulosics and modified cellulosics, starch and modified starches, xanthan gums, and guar gums.

The backbone may be an amine. Amines useful as the backbone include both mono-, di, tri-, tetra-, multi- and polymeric amines. By "amine" as used herein is meant an amine, or an amine derivative including, but not limited to, quaternized amines, N-oxides, and alkoxylated amines. These include, but are not limited to, dimethyl aminoethyl methacrylate (DMAEMA), polyethylene imideacrylamide, amino acids, bis(hexamethylene)triamine, morpholine, polyalkyleneimines, aliphatic and cycloaliphatic amines, alkoxyalkylamines, aliphatic amino alcohols, monoamino carboxylic acids,glucosamine, melamine, urea, guannidine, polyguanides, piperdine, morpholine, and derivatives thereof. Useful amines also include those that can be hydrolyzed to form a primary amine such as acetamide and n-vinyl formamide.

Finally, the backbone may consist of a molecule having both amine and hydrophobic functionality, such as a carboxylated amine or a propoxylated amine such as the Jeffamines available from Hunstman. In the case of a backbone having both amine and hydrophobic functionalities, amine-functional arms will be grafted onto the backbone.

The grafted arms consist of polymers formed from ethylenically unsaturated monomers, either hydrophobic polymers grafted onto an amine-functional backbone, or amine or amide polymers grafted onto a hydrophobic backbone. Examples of ethlenically unsaturated monomers include, but are not limited to, maleates, (meth)acrylamides, vinyl esters, itaconates, styrenics, unsaturated hydrocarbons and acrylonitrile, nitrogen functional monomers, vinyl esters, alcohol functional monomers, and (meth)acrylates.

Grafting is generally accomplished by the addition of an initiator to a solution containing the functional polymer backbone and the moiety to be grafted onto it. The initiator and reaction conditions are chosen such that the initiator is very reactive under those conditions. The activity must be such that it generates grafting sites on the backbone. This is largely dependent on the initiator, reaction temperature and the relative concentrations of the reactants. The reaction temperature and reaction conditions are chosen such that the initiator has a short half life under those conditions. Typically, the initiator will generate an active site on the backbone by abstracting a proton from the backbone leaving a free radical site on the backbone. This free radical site then reacts with the ethylenically unsaturated monomer to form the grafted molecule. One undesirable side reaction that must be minimized during the grafting reaction is the reaction of the initiator with the ethylenically unsaturated monomers to form a homopolymer or copolymer chain. Persons skilled in the art will realize that this can be achieved by judicious choices or initiators, temperature, and relative concentrations of the initiator, monomer and of the backbone moieties. Higher temperatures or the use of very reactive initiators may increase the grafted fraction in the final product. Also, the higher the ratio of the concentration of backbone and initiator to the monomer the greater is the grafting efficiency. The grafting efficiency is enhanced by minimizing the amount of solvent or diluent in the reaction. It is preferable that the backbone act as a reactant as well as a solvent. Preferred initiators for the grafting reaction include peroxides.

It is critical to the present invention to balance the level of hydrophobicity and amine functionality. The weight ratio of the hydrophobic moiety to the amine-functional moiety is from 1000:1 to 1:1000; preferably from 100:1 to 1:100; more preferably from 10:1 to 1:10; and most preferably about 1:1.

The polymers of the present invention are particularly effective at delivering actives from an aqueous medium. The graft polymer associates with the surface of a substrate, carrying one or more active ingredients with it. Substrates to which the graft copolymers will associate include, but are not limited to, textiles, fabrics, ceramics, paper, leather, wood, hair, skin, metal, tile, carpet, floor coverings, cementitious substrates, glass, plastic, non-wovens, concrete, insulation, mineral slurries, and shale.
The acitive ingredient(s) may be encapsuulated, coated, or associated with the graft polymer. The polymers may be used to deliver water-soluble hydroxy containing moieties, such as hydroxy alkyl ureas, sugar and other soluble ingredients to the surface of the fabric from a wash liquor. These water soluble additives would typically stay in the wash liquor and wash down the drain. Insoluble ingredients, such as silicone oils may be delivered to the substrate by association with the hydrophobic moiety of the graft polymer. While being not bound by theory, we believe that the hydrophilic part of the graft copolymers interacts with water soluble moieties such as glycerol through H bonding mechanisms. The hydrophobic part of the molecule tends to deposit on the fabric bringing along with it the hydrophilic part of the graft copolymer which drags the water soluble additive to the surface due to the afore mentioned interaction. Conversely, the polymer may be used to disperse a completely water insoluble molecule such as perfumes and silicones in an aqueous medium. The interaction between the hydrophobic part of the graft copolymer then delivers these water insoluble actives to the surface of the fabric in the wash liquor. The same mechanism can be used to deliver actives in personal care applications such as shampoos, conditioners, bath additives etc. The additives do not necessarily have to be encapsulated by the graft copolymers. The strong interaction of these polymers with the afore mentioned additives is sufficient to deliver these additives to the desired surfaces even from very dilute aqueous media.

The amide graft polymers also have an affinity for substrates. They are especially effective in associating with hard surfaces such as ceramics, tile and flooring. While not being bound by any particular theory, it is believed that the association is related to the nucleophilic nature of the electron pair on the nitrogen atom. This nucleophilic nature is even greater with substituted amides, which are preferred.

The polymers of the present invention are pH triggerable due to the amine functionality. This property make them useful in control release applications. While not being bound to any particular theory, it is believed that the polymers are insoluble at high pH due to the hydrophobic portion of the molecule. As the pH of the environment is lowered, the amine moieties become protonated, making the polymer more soluble.

Active substances can be encapsulated by the graft molecule of the present invention by means known in the art such as spray-drying. Examples of useful active components include, but are not limited to perfumes, enzymes, fabric softener molecules, bleaches such as percarbonates and perborates, rinse aid additives, bleach activators such as TAED and NOBS, optical brightener. In a detergent application, the encapsulated active ingredient is insoluble in the wash cycle, where the pH is generally 8 to 11. In the rinse cycle, where the pH drops to about 7 to 8, the active is released to combine with the clothes.

The graft copolymers can be used in a variety of aqueous cleaning applications such as fabric (home, institutional and industrial), autodish, hard surfaces and the processing of textiles. In fabric care the graft copolymer may be incorporated in to detergent formulations which are in the form of powders, liquids, tablets, etc.

The graft polymer of the present invention can also be used in non-aqueous systems, such as in detergent sachets containing a non-aqueous mixture which are release by dissolution of the sachet. Also, since most of these polymers can be made without using a solvent such as water, alcohols, glycol etc, these molecules are particularly useful in non-aqueous applications such as inkjet printing, dry cleaning systems, deicing fluids, dispersing hydrophobic pigments etc.

The pH trigger can be tailored to the application by adjustment of the hydrophobic/amine ratio. Thus the trigger can be made to operate at any desired pH.

The soluble/insoluble pH trigger can be shifted by a change in temperature. Thus a graft molecule can be designed for an autodish application having a wash temperature in the range of 40-60°C and a wash pH, and a rinse temperature in the range of 10-30°C but a pH lower than the wash pH.

The amine or amide hydrophobe graft polymers of the present invention are also useful in washing machine detergents and fabric softeners where they also function as color protective agents, anti pilling agents and antiwrinkle agents; in anti-wrinkle spray applications, in autodish applications where they minimize the filming and spotting on dishware, in hard surface cleaners where they improve cleaning and extend the time interval between cleanings, in metal working fluids, as corrosion inhibitors, as additives in paper coatings to minimize bleeding of dyes, in ink jet printing ; as textile finishing aids where they function to improve abrasion resistance; medical applications; personal care applications such as a shampoo/conditioner where the encapsulated conditioner is insoluble in basic shampoo, yet is released during rinse ; for binders in non-woven applications; for shale stabilizers in oilfield applications where they are attracted to the anionic shale surface to fill holes, and as additives that minimize water but maximize oil production.

The following examples are presented to further illustrate and explain the present invention and should not be taken as limiting in any regard.

### Example 1: Grafting DMAEMA on to poly(propylene glycol).

A mixture of 100 grams of isopropyl alcohol and 100 grams of poly(propylene glycol) Mw=3000 was heated to 83 to 85C. 100 grams of dimethyl amino ethyl methacrylate (DMAEMA) (Ageflex FM1 obtained from Ciba) was added over a period of an hour. 2.0 grams of tertiary butyl peroctoate (Esperox 28 from Elf Atochem) dissolved in 30 grams of isopropanol was simultaneously added over a period of 1 hour and 30 minutes. The clear yellow product was heated at that temperature for 2 hours. 38.2 grams of acetic acid in 400 grams of water were then added. The reactor was set up for distillation and 2 drops of ANTIFOAM were added to ease the distillation process by minimizing foaming. About 125 grams of distillate was collected with the final product being an opaque but homogenous liquid.

### Example 2: Grafting methyl acrylate on to poly(ethylene imine).

100 grams of isopropyl alcohol, 100 grams of deionized water and 34 grams of acetic acid were added to a reactor. 100 grams of poly(ethylene imine) (Lupasol PS from BASF) were then added to the reactor. The mixture was heated to 80C. 100 grams of methyl acrylate was added to the reactor over a period of 1 hour. 3.0 grams of sodium persulfate dissolved in 50 grams of deionized water were added over a period of 1.5 hours. The reaction product was heated for 2 hours at 80C and was an opaque amber solution at that point. 16.2 grams of acetic acid and 300 grams of deionized water was then added and the reactor set up for distillation. Approximately 135 grams of distillate was removed and the final product was a homogenous opaque yellowish brown solution.

### Example 3: Grafting methyl acrylate on to Kymene R201.

105 grams of isopropyl alcohol, 100 grams of deionized water and 12.5 grams of acetic acid were added to a reactor. 100 grams of a polymeric amine (Kymene R201 from Hercules) were then added to the reactor. The mixture was heated to 85C. 100 grams of methyl acrylate was added to the reactor over a period of 1 hour. 3.0 grams of sodium persulfate dissolved in 50 grams of deionized water were added over a period of 1.5 hours. The reaction product was heated for 2 hours at 80C and was an opaque yellow solution at that point. 24.0 grams of acetic acid and 300 grams of deionized water was then added and the reactor set up for distillation. Approximately 175 grams of distillate was removed and the final product was an opaque yellowish white solution.

### Example 4: Grafting DMAEMA onto Jeffamine T-5000.

100 grams of isopropyl alcohol was added to a polymerization reactor. 100 grams of a glyceryl poly(oxy propylene) triamine (Jeffamine T-5000) was then added to the reactor. The mixture was heated to 85C. 100 grams of dimethyl amino ethyl methacrylate (DMAEMA) (Ageflex FM1 obtained from Ciba) was added over a period of an hour. 2.0 grams of tertiary butyl peroctoate (Esperox 28 from Elf Atochem) dissolved in 30 grams of isopropanol was simultaneously added over a period of 1 hour and 30 minutes. The clear yellow product was heated at that temperature for 2 hours. 38.2 grams of acetic acid in 350 grams of water were then added. The reactor was set up for distillation and 2 drops of ANTIFOAM were added to ease the distillation process by minimizing foaming. About 190 grams of distillate was collected with the final product being an opaque but homogenous liquid.

### Example 5: Grafting DMAEMA onto an alcohol ethoxylate.

100 grams of isopropyl alcohol was added to a polymerization reactor. 100 grams of a C12 to 15 alcohol ethoxylate with 9 moles of ethylene oxide (Neodal 25-9 from Shell) was then added to the reactor. The mixture was heated to 85C. 100 grams of dimethyl amino ethyl methacrylate (DMAEMA) (Ageflex FM1 obtained from Ciba) was added over a period of an hour. 2.0 grams of tertiary butyl peroctoate (Esperox 28 from Elf Atochem) dissolved in 30 grams of isopropanol was simultaneously added over a period of 1 hour and 30 minutes. The clear yellow product was heated at that temperature for 2 hours. 38.2 grams of acetic acid in 400 grams of water were then added. The reactor was set up for distillation and 2 drops of ANTIFOAM were added to ease the distillation process by minimizing foaming. About 175 grams of distillate was collected. The final product was opaque when hot but formed a clear yellow homogenous solution on cooling.

### Example 6: Grafting DMAEMA onto an alcohol ethoxylate.

100 grams of isopropyl alcohol was added to a polymerization reactor. 100 grams of a C12 to 13 alcohol ethoxylate with 5 moles of ethylene oxide (Neodal 25-9 from Shell) was then added to the reactor. The mixture was heated to 85C. 100 grams of dimethyl amino ethyl methacrylate (DMAEMA) (Ageflex FM1 obtained from Ciba) was added over a period of an hour. 2.0 grams of tertiary butyl peroctoate (Esperox 28 from Elf Atochem) dissolved in 30 grams of isopropanol was simultaneously added over a period of 1 hour and 30 minutes. The clear yellow product was heated at that temperature for 2 hours. The reactor was set up for distillation and approximately 22 grams of distillate was collected. The neat non-aqueous product may be used by itself. This is especially beneficial for non-aqueous formulations in which water is a detriment to stability. However, the product may be neutralized with an acid and diluted with water.

### Example 7: Grafting DMAEMA onto an amine oxide surfactant.

50 grams of isopropyl alcohol was added to a polymerization reactor. 50 grams of a C16 amine oxide (Ammonyx CO from Stepan) was then added to the reactor. The mixture was heated to 85C. 50 grams of dimethyl amino ethyl methacrylate (DMAEMA) (Ageflex FM1 obtained from Ciba) dissolved in 50 grams of Dl water was added over a period of an hour. 2.0 grams of tertiary butyl peroctoate (Esperox 28 from Elf Atochem) dissolved in 30 grams of isopropanol was simultaneously added over a period of 1 hour and 30 minutes. The clear yellow product was heated at that temperature for 2 hours. The reactor was set up for distillation and approximately 85 grams of distillate was collected. The neat non-aqueous product may be used by itself. This is especially beneficial for non-aqueous formulations in which water is a detriment to stability. However, the product may be neutralized with an acid and diluted with water.

### Example 8: Grafting DMAEMA onto a cationic surfactant.

200 grams of cetytrimethyl ammonium chloride 25% solution in water (available from Aldrich) was added to a polymerization reactor and heated to 85C. 50 grams of dimethyl amino ethyl methacrylate (DMAEMA) (Ageflex FM1 obtained from Ciba) dissolved in 50 grams of Dl water was added over a period of an hour. 7.0 grams of 35% hydrogen peroxide dissolved in 35 grams of deionized water was simultaneously added over a period of 1 hour and 30 minutes. 1.9 grams of Bruggolite FF6 (available from Bruggeman Chemical) in 38 grams deionized water was added over the same period of time. The clear yellow product was heated at that temperature for 2 hours.

### Example 9: Grafting t-butylaminoethyl methacrylate onto an alcohol ethoxylate.

100 grams of a C9 to 11 alcohol ethoxylate with 2.5 moles of ethylene oxide (Neodal 91-2.5 from Shell) was introduced in to a polymerization reactor. The surfactant was heated to 90C. 100 grams t-butylaminoethyl methacrylate (Ageflex FM4 obtained from Ciba) was added over a period of an hour and 15 minutes. 2.0 grams of tertiary butyl peroctoate (Esperox 28 from Elf Atochem) dissolved in 30 grams of Neodol 91-2.5 was simultaneously added over a period of 1 hour and 30 minutes. The clear yellow product was heated at that temperature for 2 hours. The final product was a clear yellow homogenous solution on cooling.

### Example 10: Grafting dimethylacrylamide on to an alcohol ethoxylate.

487 grams of a C12 to 13 alcohol ethoxylate with 5 moles of ethylene oxide (Neodal 23-5 from Shell) was introduced in to a polymerization reactor. The surfactant was heated to 90C. 487 grams of dimethylacrylamide (available from Kohjin in Japan) was added over a period of 5 hours. 9.8 grams of tertiary butyl peroctoate (Esperox 28 from Elf Atochem) dissolved in 146 grams of Neodol 23-5 was simultaneously added over a period of 5 hours and 15 minutes. The clear yellow product was heated at that temperature for 2 hours. The final product was a clear yellow viscous homogenous solution on cooling.

### Example 11: Grafting DMAEMA on to an EO-PO block copolymer.

100 grams of Pluronic L-61 (an EO-PO block copolymer available from BASF) was introduced in to a polymerization reactor. The block copolymer was heated to 90C. 100 grams of DMAEMA(Ageflex FM1 available from Ciba) was added over a period of 1 hours. 2.0 grams of tertiary butyl peroctoate (Esperox 28 from Elf Atochem) dissolved in 30 grams of Neodol 91-2.5 was simultaneously added over a period of 1.5 hours. The opaque yellow product was heated at that temperature for 2 hours.

### Example 12: Grafting dimethylacrylamide on to a branched poly(propylene glycol).

170 grams of Jeffamine T403 (a three arm polypropylene glycol available from Huntsman) was introduced in to a polymerization reactor. The surfactant was heated to 90C. 100 grams of dimethylacrylamide (available from Kohjin in Japan) was added over a period of 1.75 hours. 2.0 grams of tertiary butyl peroctoate (Esperox 28 from Elf Atochem) dissolved in 30 grams of Jeffamine T403 was simultaneously added over a period of 2 hours. The clear yellow product was heated at that temperature for 3 hours. The final product was a clear yellow viscous homogenous solution on cooling.

### Example 13: Grafting diethylaminoethyl methacrylate on to a EO-PO copolymer.

170 grams of Jeffamine M-6000 (a PO/EO 9/1 copolymer available from Huntsman) was introduced in to a polymerization reactor. The material was heated to 90C. 100 grams of diethylaminoethyl methacrylate (Ageflex FM2 from Ciba) was added over a period of 1.25 hours. 2.0 grams of tertiary butyl peroctoate (Esperox 28 from Elf Atochem) dissolved in 30 grams of Jeffamine M-6000 was simultaneously added over a period of 1.5 hours. The clear yellow product was heated at that temperature for 3 hours. The final product was a clear yellow viscous homogenous solution on cooling.

### Example 14: Encapsulation of an active (enzyme).

The polymer of Example 1 could be sprayed on to an enzyme. The encapsulated enzyme can then be used in a fabric care detergent formulation.

### Example 15:

The polymer of Example 1 could be added to a liquid fabric softener formulation. The formulation with the polymer had better pill reduction properties than the fabric softener by itself.

### EXAMPLE 16

### Granular Detergent Test Composition Preparation

Typical heavy duty granular detergent compositions can be prepared containing one or more polymers of this invention. These granular detergent compositions all have the following basic formula:

**TABLE I**

| **Component** | **Wt. %** |
|---|---|
| C₁₂ Linear alkyl benzene sulfonate | 9.31 |
| C₁₄₋₁₅ alkyl ether (0.35 EO) sulfate | 12.74 |
| Zeolite Builder | 27.79 |
| Sodium Carbonate | 27.31 |
| PEG 4000 | 1.60 |
| Dispersant | 2.26 |
| C₁₂₋₁₃ Alcohol Ethoxylate (9 EO) | 1.5 |
| Sodium Perborate | 1.03 |
| Soil Release Polymer | 0.41 |
| Enzymes | 0.59 |
| Polymer of Example 1 | 3.0 |
| Perfume, Brightener, Suds Suppressor, Other Minors, Moisture, Sulfate | Balance |
| | 100% |

### EXAMPLE 17

### Liquid Detergent Test Composition Preparation

Typical heavy duty liquid detergent compositions can be prepared containing the polymers of this invention. These granular detergent compositions all have the following basic formula:

**TABLE II**

| **Component** | **Wt. %** |
|---|---|
| C12-15 alkyl ether (2.5) sulfate | 38 |
| C12 glucose amide | 6.86 |
| Citric Acid | 4.75 |
| C12-14 Fatty Acid | 2.00 |
| Enzymes | 1.02 |
| MEA | 1.0 |
| Propanediol | 0.36 |
| Borax | 6.58 |
| Dispersant | 1.48 |
| Na Toluene Sulfonate | 6.25 |
| Polymer of Example 6 | 1.0 |
| Dye, Perfume, Brighteners, Preservatives, Suds Suppressor, Other Minors, Water | Balance |
| | 100% |

### EXAMPLE 18

### Granular Detergent Test Composition Preparation

Typical granular detergent compositions can be prepared containing polymers of this invention. These granular detergent compositions all have the following basic formula:

**TABLE III**

| | **Example** | **Comparative** |
|---|---|---|
| **Component** | **Wt. %** | **Wt. %** |
| Na C₁₂ Linear alkyl benzene sulfonate | 9.40 | 9.40 |
| Na C₁₄₋₁₅ alkyl sulfonate | 11.26 | 11.26 |
| Zeolite Builder | 27.79 | 27.29 |
| Sodium Carbonate | 27.31 | 27.31 |
| PEG 4000 | 1.60 | 1.60 |
| Dispersant, Na polyacrylate | 2.26 | 2.26 |
| C₁₂₋₁₃ alkyl ethoxylate (E9) | 1.5 | 1.5 |
| Sodium Perborate | 1.03 | 1.03 |
| Polymer of Example 2 | 2.0 | 0 |
| Other Adjunct ingredients | Balance | Balance |
| | 100% | 100% |
| | | |

### Example 19

Typical dilute fabric softener formulations are listed below.

**TABLE 4**

| **Formulations of Dilute Traditional Softeners (Single Active)**^{**a**} | |
|---|---|
| **Formula A** | |
| **Ingredient** | **(%)** |
| distearyldimethylammonium Chloride (75% | 6-9 |
| active) | 0.1 - 3.0 |
| Polymer of Example 3 | 0.2-0.5 |
| Perfume | 0.001 |
| Colorant | Balance |
| Water | |

| **Formula B** | |
|---|---|
| Quaternary dialkylimidazolines (75% active) | 6-9 |
| Polymer of Example 4 | 0.1 - 3.0 |
| Perfume | 0.2-0.5 |
| Colorant | 0.001 |
| Preservative | + |

### Example 20: Examples of concentrated fabric softener compositions.

**TABLE 5**

| **Ready-to-Use Rinse Conditioners at Triple Concentration (Mixed Actives)**^{**a**} | |
|---|---|
| **Formula C** | |
| **Ingredient** | **(%)** |
| distearyldimethylammonium chloride 75% | 14 |
| Polymer of Example 6 | 3 |
| Lanolin | 2 |
| Ethoxylated fatty acid | 4 |
| CaCl₂ | 0.05 |
| Water, perfume, color | Balance |

| **Fomulation D** | |
|---|---|
| distearyldimethylammonium chloride | 5-10 |
| Amidoamine | 5-10 |
| Imidazoline | 3.75-5.25 |
| Polymer of Example 7 | 0.1 - 2.0 |
| Electrolyte | 0.05-.4 |
| Water, perfume, color | Balance |
| | |

### Example 21:

Some of the polymers synthesized in Examples 1 to 8 were evaluated for pill reduction and color protection properties in detergent formulations. The tests were conducted in a full scale washing machine using 95.5g/load Ultra Xtra liquid detergent and 1% polymer. The wash used City H₂O, at 93°F, with a 10 minute wash. The load contained 3 black knit & 3 red knit swatches (obtained from Textile Innovators) attached to one pillowcase and 8 extra pillow cases added as ballast. The test was conducted through 5 cycles.

The interlock knit swatches used in the test were evaluated on the spectrophotometer to measure any evidence of color protection. The results of this evaluation appear in the table below. The delta E indicates the difference between the color of a new swatch compared to that of one that had been through five wash cycles under the following conditions:

**TABLE 6**

| Sample | Sample description | Delta E* (compared to new swatch) | |
|---|---|---|---|
| | | Red | Black |
| Control (no polymer) | - | 4.01 | 6.67 |
| Polymer of Example 2 | Methyl acrylate - g -polyethyleneimine (50 wt%) | 3.28 | 1.28 |
| Polymer of Example 4 | DMAEMA-g-Jeffamine T-5000 | 4.5 | 1.8 |
| Polymer of Example 1 | DMAEMA-g-poly(propylene glycol) (50 wt%) | 4.71 | 1.81 |

The lower delta E numbers indicate improved color protection or less dye less over the control. The swatches were visually evaluated for pilling.

**TABLE 7**

| Sample | | Pilling Evaluation On a scale of 1 to 5 (5 being the best) |
|---|---|---|
| Control (no polymer) | - | 1 |
| Polymer of Example 2 | Methyl acrylate - g polyethyleneimine | 3 |
| Polymer of Example 4 | DMAEMA-g-Jeffamine T-5000 | 4 |
| Polymer of Example 1 | DMAEMA-g-poly(propylene glycol) | 5 |

The data indicate that the Polymers of this invention have excellent pill reduction properties.

### Example 22:

The polymer of Example 1 was evaluated for soil anti-redeposition interference by running a full-scale anti-redeposition test. Test conditions were as follows:

| |
|---|
| 97g/Load Sam's Choice liquid |
| 1% polymer |
| 30 grams of bandy black clay, 150ppm H₂O, 93°F, regular cycle |
| The wash load contained 3 poly/cotton swatches & 3 cotton swatches with approximately 3 Ibs of ballast. |

**TABLE 8**

| Sample | Delta Whiteness Index | |
|---|---|---|
| | Cotton | Poly/cotton |
| Control | 32.88 | 10.28 |
| Polymer of Example 1 | 6.87 | 15.18 |

The data indicate that the polymer actually improves the anti-redeposition properties on cotton.

### Example 23: Solubility at different pH.

The polymer of Example 4 was diluted to a 0.5% solution. This solution was adjusted to various pH's using 50% caustic. The solubility data is listed in Table 9 below.

**TABLE 9**

| PH of the solution | Solubility of the polymer |
|---|---|
| 4.8 | Soluble |
| 8.0 | Soluble |
| 11.7 | Insoluble |
| | |

### Example 24. Personal Care formulation

| Water repellant sunscreen | |
|---|---|
| Ingredients | Wt% |
| Glycerin | 5.0 |
| Polymer of Example 7 | 2.0 |
| PEG 100 stearate | 5.0 |
| Isostearyl stearate | 4.0 |
| Octyl methoxycinnamate | 7.5 |
| Butyl methoxydibenzoylmethane | 1.5 |
| Hexyl methicone | 5.0 |
| Dl water | rest |

### Example 25. Textile application

The polymer of Example 4 was padded on to cotton fabric during the textile finishing process. The weight of the polymer put on to the fabric was 1 weight percent by weight of the fabric. The treated and finished fabric was then run through 25 cycles of a regular washing machine. The treated fabric exhibited less dye loss and wear and tear as compared to an untreated fabric.

### Example 26. Textile application

The polymer of Example 8 was included in a process of stone washing of denim fabrics. The process involves removal of indigo dye from denim using pumice stones (for mechanical action) and cellulase enzymes (chemical action). The stone washed denim using polymer 2 had significantly less redeposition of the indigo (usually called backstaining) than a control which did not contain the polymer.

### Example 27. Typical Hard surface cleaning formulations

| Acid Cleaner | |
|---|---|
| Ingredient | wt% |
| Citric acid (50% solution) | 12.0 |
| C12-15 linear alcohol ethoxylate with 3 moles of EO | 5.0 |
| Alkylbenzene sulfonic acid | 3.0 |
| Polymer of Example 10 | 1.0 |
| Water | 79.0 |

| Alkaline Cleaner | |
|---|---|
| Water | 89.0 |
| Sodium tripolyphosphate | 2.0 |
| Sodium silicate | 1.9 |
| NaOH (50%) | 0.1 |
| Dipropylene glycol monomethyl ether | 5.0 |
| Octyl polyethoxyethanol, 12-13 moles EO | 1.0 |
| Polymer of example 4 | 1.0 |

### Example 28 Typical automatic dishwash formulation

| Ingredients | Amounts |
|---|---|
| Sodium tripolyphosphate | 25.0 |
| Sodium carbonate | 25.0 |
| C12-15 linear alcohol ethoxylate with 7 moles of EO | 3.0 |
| Polymer of Example 7 | 4.0 |
| Sodium sulfate | rest |

### Example 29 Car wash rinse off aid formulation

| Ingredients | wt% |
|---|---|
| Water | 80 |
| Butyldiglycol | 10 |
| Polymer of Example 2 | 10 |

### Example 30 Deposition of fragrances during wash cycles

A fruity masking fragrance (0.5%) was emusified in water using the polymer of Example 7 using a high shear mixer. This emulsified fragrance was then run through a typical wash cycle using 1.0 g/liter of Arm and Hammer Free (free of fragrances) and 0.5% fragrance. The washed swatches were then evaluated by a panel. The results of the panel are listed in the Table 10.

**TABLE 10**

| | |
|---|---|
| Polymer | Average results of the Panel |
| Control | - |
| Polymer 7 | More fragrant than the control |

### Example 31: Use of these polymers in anti-wrinkle applications.

The polymers of this invention were tested for anti-wrinkle in the main wash. The tests were conducted as follows: The tests were conducted over 2 cycles, but no drying in between. The test used 58g Tide powder and the wash had five 10" x 10" prewashed TIC 400 bleached print cloth with 12 ballast pillowcases and 3 ballast towels. The wash was 10 minute wash @ 93°F using 70 ppm hardness water. The swatches were allowed to hang dry. The swatches were then visually evaluated using the following grading scheme:
1 - same or worse than control
2 - slightly better than control
3 - better than the control
4 - much better than the control

The test was used to try and deposit a water soluble additive N-hydroxyethyl urea which should improve anti-wrinkling. The data from the test is listed below.

| | | |
|---|---|---|
| Anti-wrinkling using | Anti-wrinkling using | Anti-wrinkling using |
| 2 wt% N-hydroxyethyl | 2 wt% Polymer of | 1 wt% of Polymer of |
| urea based on weight of | Example 8a based on | Example 8a and 1% N- |
| detergent | weight of detergent | hydroxyethyl urea |
| | | based on weight of |
| | | detergent |
| 1 | 1 | 4 |

The data indicate that the water soluble additive or the polymer by itself has no anti-wrinkling properties but the combination of the two does. This indicates that the polymer will deposit a water soluble additive such as N-hydroxyethyl urea on to the fabric.

## Claims

1. A graft polymer comprising a backbone moiety and at least one moiety grafted onto the backbone moiety, wherein said backbone and grafted moieties comprise a hydrophobe and an amine or amide.

2. The graft polymer of claim 1 comprising a hydrophobic backbone and a grafted moiety comprising an amine, an amide, or a mixture thereof.

3. The graft copolymer of claim 2, wherein said hydrophobic backbone is a polymer

4. The graft copolymer of claim 3 wherein said hydrophobic backbone polymer is a natural polymer.

5. The graft copolymer of claim 2 wherein said hydrophobic backbone is a non-polymer.

6. The graft polymer of claim 1 comprising an amine backbone and a grafted hydrophobic moiety.

7. The graft polymer of claim 1 comprising a backbone moiety consisting of both a hydrophobic moiety and an amine moiety, wherein said grafted moiety is an amine, amide, or mixture thereof.

8. The graft copolymer of claim 1 wherein said amine comprises a quaternized amine, N-oxide, alkoxylated amine, or mixture thereof.

9. The graft copolymer of claim 1 wherein said amine is at least partially neutralized.

10. The graft polymer of claim 1 wherein the weight ratio of said amine to said hydrophobe is from 1000:1 to 1:1000.

11. The graft polymer of claim 10 wherein the weight ratio of said amine to said hydrophobe is from 100:1 to 1:100.

12. The graft polymer of claim 11 wherein the weight ratio of said amine to said hydrophobe is from 10:1 to 1:10.

13. The graft polymer of claim 11 wherein the weight ratio of said amine to said hydrophobe is about 1:1.

14. A composition comprising the graft polymer of claim 1, and an ingredient selected from the group consisting of a surfactant, builder, enzyme, perfume, optical brightner, fillers, anti-fungal and antimicrobial agents, pigments, co-builders, anti-oxidants, dispersants, anti-foaming agents, acids, bases, preservatives, water-softening agents, sunscreen agents and mixtures thereof.

15. A treated substrate comprising a substrate having associated thereon the graft copolymer of claim 1.

16. The treated substrate of claim 15, wherein said substrate is selected from the group consisting of textiles, fabrics, ceramics, paper, leather, wood, hair, skin, metal, tile, carpet, floor coverings, cementitious substrates, glass, plastic, non-wovens, concrete, insulation, mineral slurries, and shale

17. A control release formulation comprising an active substance encapsulated, coated, or associated with the graft copolymer of claim 1.

18. The control release formulation of claim 17, wherein said active substance comprises at least one water-soluble substance, at least one water-insoluble substance, or a mixture thereof.

19. A method for the delivery of an active ingredient comprising:
a) coating or encapsulating an active ingredient with the graft copolymer of claim 1;
b) introducing said coated or encapsulated active ingredient into an aqueous environment; and
c) decreasing the pH of the aqueous environment to solubilize the graft copolymer, thereby releasing the active ingredient.

20. The method of claim 19 wherein the aqueous environment of step b) has a pH of greater than pH 8, and wherein the pH of said aqueous environment is lowered below pH 8 in step c).
